# EUROPEAN PATENT SPECIFICATION

(11) **EP 3 652 143 B1**
(45) Date of publication and mention of the grant of the patent: **03.08.2022**
(21) Application number: 18734846.1
(22) Date of filing: 06.07.2018
(51) Int. Cl.: C07C 69/82, C08G 63/00

(54) **PROCESS FOR THE PRODUCTION OF BIS-HYDROXYALKYLENE DICARBOXYLATES**
VERFAHREN ZUR HERSTELLUNG VON BIS-HYDROXYALKYLENDICARBOXYLATEN
PROCÉDÉ DE PRODUCTION DE DICARBOXYLATES BIS-HYDROXYALKYLÈNE

(30) Priority: 12.07.2017 EP 17180929
(43) Date of publication of application: 20.05.2020
(73) Proprietor: SABIC Global Technologies B.V., 4612 PX Bergen op Zoom (NL)
(72) Inventor: ALIVE, Keshavaraja, 6160 GA Geleen (NL); ALIDEDEOGLU, Husnu, Alp, 6160 GA Geleen (NL); KUMAR, Prashant, 6160 GA Geleen (NL)
(74) Representative: Sabic Intellectual Property Group
(86) International application number: PCT/EP2018/068406
(87) International publication number: WO 2019/011816

(56) References cited:
- CN-A- 106 478 931
- CN-A- 106 519 196
- DATABASE CA [Online] CHEMICAL ABSTRACTS SERVICE, COLUMBUS, OHIO, US; SHAPIROVSKII, YU. A. ET AL: "Glycol esters of terephthalic acid, monomers for poly(ethylene terephthalate)", XP002773788, retrieved from STN Database accession no. 1973:515908 & SHAPIROVSKII, YU. A. ET AL: "Glycol esters of terephthalic acid, monomers for poly(ethylene terephthalate)", TR. VSES. NAUCH.-ISSLED. PROEKT. INST. MONOMEROV , 3(3), 42-8 FROM: REF. ZH., KHIM. 1973, ABSTR. NO. 5N192, 1972,

## Description

The present invention relates to a process for the production of bis-hydroxyalkylene dicarboxylates. In particular, the invention relates to an improved process for the production of bis-hydroxyethylene tererphthalate (BHET) and bis-hydroxybutylene terephthalate (BHBT).

Bis-hydroxyalkylene dicarboxylates are well-known chemical compounds, notably used as intermediate product in the production of polyesters such as for example poly(ethylene terephthalates) (PET) and poly (butylene terephthalates) (PBT).

In conventional processes, such as operated in polyester production processes according to the state of the art, bis-hydroxyalkylene dicarboxylates are commonly produced using tetraalkyl-substituted titanate catalysts. For example, tetrabutyl titanate and tetraisopropyl titanate are commonly used as catalysts. CN 106519196 A discloses a process for the preparation of high quality polybutylene terephthalate wherein tetrabutyl titanate is used as the catalyst.

However, processes for the production of bis-hydroxyalkylene dicarboxylates using such conventional catalysts take a certain time to completion. It will be understood that in commercial operations, there is an ongoing desire to reduce reaction time of production processes, thereby improving the process economics and optimising the asset utilisation, leading to an increase of productivity. This also applies to processes for the production of polyesters, where the process for the production of bis-hydroxyalkylene dicarboxylates commonly forms part of. For that reason, there is an ongoing desire to have access to a process for the production of bis-hydroxyalkylene dicarboxylates with a reduced reaction time.

This has now been achieved according to the present invention by a process for the production of bis-hydroxyalkylene dicarboxylates comprising the steps in this order of:
(a) providing to a reaction vessel a reaction mixture comprising a dicarboxylic acid or a diester thereof and an alkylene diol;
(b) providing a quantity of a catalyst to the reaction mixture; and
(c) reacting the reaction mixture at a temperature in the range of ≥ 180 and ≤ 280°C
wherein the catalyst is a compound according to the formula (I): 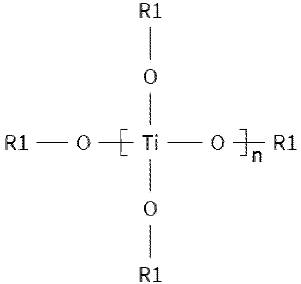 in which R1 is an alkyl moiety comprising 2-10 carbon atoms and n is an integer of ≥ 2.

Such process results in a reduction of the reaction time. It is believed that such catalyst demonstrates a higher hydrolytic stability, and thus is more resistant to loss of catalytic activity resulting from the formation of water as by-product during the reaction of the dicarboxylic acid and the alkylene diol.

In the catalyst according to formula (I), each R1 may for example be -CH₂-CH₃, -CH₂-CH₂-CH₃, -CH(CH₃)-CH₃, -CH₂-CH₂-CH₂-CH₃, or -C(CH₃)₃. Preferably, each R1 is -CH(CH₃)-CH₃, -CH₂-CH₂-CH₂-CH₃, or -C(CH₃)₃. It is particularly preferred that each R1 is -CH₂-CH₂-CH₂-CH₃.

Each R1 may be the same. Each R1 may be selected from ethyl, propyl, isopropyl, butyl or isobutyl. Preferably, each R1 is the same and selected from isopropyl, butyl or isobutyl. More preferably, each R1 is a butyl moiety.

It is preferred that n is an integer of ≥ 4 and ≤ 100. More preferably, n is an integer of ≥ 5 and ≤ 50, even more preferably ≥ 5 and ≤ 20.

The catalyst that is used in the process according to the present invention may for example be selected from poly(tetraethyl titanate), poly(tetrabutyl titanate) or poly(tetraisopropyl titanate). Preferably, the catalyst is poly(tetrabutyl titanate).

The catalyst may for example be provided to the reaction mixture in a quantity of 50 - 1000 ppm with regard to the total weight of the dicarboxylic acid or diester thereof and the alkylene diol in the reaction mixture. Preferably, the catalyst is provided in a quantity of 100 - 500 ppm, more preferably 100 - 300 ppm.

The dicarboxylic acid or a diester thereof may for example comprise 4-20 carbon atoms. The dicarboxcylic acid or diester thereof may for example be an aromatic dicarboxylic acid or diester thereof, preferably an aromatic dicarboxylic acid or diester thereof comprising 4-20 carbon atoms. The dicarboxylic acid or diester thereof may for example be selected from terephthalic acid, dimethyl terephthalate, isophthalic acid, 2,6-naphthalenedicarboxylic acid, dimethyl-2,6-naphthalenedicarboxylate, 2,5-furandicarboxylic acid, dimethyl-2,5-furandicarboxylate, or succinic acid. The dicarboxylic acid or diester thereof may for example be a mixture of compounds selected from terephthalic acid, dimethyl terephthalate, isophthalic acid, 2,6-naphthalenedicarboxylic acid, dimethyl-2,6-naphthalenedicarboxylate, 2,5-furandicarboxylic acid, dimethyl-2,5-furandicarboxylate, or succinic acid. The dicarboxylic acid or diester thereof may for example be a mixture of compounds comprising ≥ 95 wt% with regard to the total weight of the dicarboxylic acid or diester thereof of terephthalic acid and one or more further dicarboxylic acid selected from isophthalic acid, 2,6-naphthalenedicarboxylic acid, 2,5-furandicarboxylic acid and succinic acid, where the total weight of the terephthalic acid and the further dicarboxylic acid equals 100 wt% of the weight of the dicarboxylic acid or diester thereof. Preferably, the dicarboxylic acid or diester thereof is terephthalic acid, isophthalic acid or 2,5-furandicarboxylic acid. More preferably, the dicarboxylic acid or diester thereof is terephthalic acid.

The alkylene diol may for example be an alkylene diol comprising 2-10 carbon atoms. The alkylene diol may for example be an aliphatic alkylene diol, preferably an aliphatic alkylene diol comprising 2-10 carbon atoms. The alkylene diol may for example be selected from ethylene glycol, propanediol, 1,4-butanediol, or cyclohexanedimethanol. Preferably, the alkylene diol is ethylene glycol or 1,4-butanediol. More preferably, the alkylene diol is 1,4-butanediol.

It is preferred that the dicarboxylic acid or a diester thereof is selected from terephthalic acid, dimethyl terephthalate, isophthalic acid, 2,6-naphthalenedicarboxylic acid, dimethyl-2,6-naphthalenedicarboxylate, 2,5-furandicarboxylic acid, dimethyl-2,5-furandicarboxylate, or succinic acid, and the alkylene diol is selected from ethylene glycol, propanediol, 1,4-butanediol, or cyclohexanedimethanol. It is particularly preferred that the dicarboxylic acid or diester thereof is selected from terephthalic acid, isophthalic acid or 2,5-furandicarboxylic acid, and the alkylene diol is selected from ethylene glycol or 1,4-butanediol. Further particularly, it is preferred that the dicarboxylic acid or diester thereof is terephthalic acid, and the alkylene diol is selected from ethylene glycol or 1,4-butanediol. Preferably, the dicarboxylic acid or diester thereof is terephthalic acid and the alkylene diol is 1,4-butanediol. Alternatively, the dicarboxylic acid or diester thereof is terephthalic acid and the alkylene diol is ethylene glycol. Alternatively, the dicarboxylic acid or diester thereof is 2,5-furandicarboxylic acid and the alkylene diol is ethylene glycol.

It is preferred that the alkylene diol is present in the reaction mixture in an excess molar ratio compared to the dicarboxylic acid or diester thereof. The molar ratio of the alkylene diol to the dicarboxylic acid or diester thereof in the reaction mixture may for example be ≥ 1.1. Preferably, the molar ratio is ≥ 1.5, even more preferable ≥ 2.0. The molar ratio of the alkylene diol to the dicarboxylic acid or diester thereof in the reaction mixture may for example be ≤ 4.0. Preferably, the molar ratio is ≤ 3.5, more preferably ≤ 3.0.

It is particularly preferred that the molar ratio of the alkylene diol to the dicarboxylic acid or diester thereof in the reaction mixture is ≥ 1.1 and ≤ 4.0, even more particularly ≥ 1.1 and ≤ 3.5, for example ≥ 2.0 and ≤ 3.0.

The reaction of the reaction mixture in the process of the present invention takes place at a temperature in the range of ≥ 200 and ≤ 280°C. Preferably, the temperature is in the range of ≥ 220 and ≤ 270°C, or ≥ 230 and ≤ 260°C.

The reaction product obtained from the process of the present invention may for example be used in a process for the production of polyesters. Such polyesters may for example be poly(ethylene terephthalate), poly(ethylene naphthalate), poly(trimethylene terephthalate), poly(ethylene furanoate), or poly(butylene terephthalate). For example, the polyester may be poly(butylene terephthalate).

The production of such polyester may for example be performed using the reaction product of the process for the production of bis-hydroxyalkylene dicarboxylates according to the invention. The production of polyester may for example be performed by subjecting the reaction product comprising the bis-hydroxyalkylene dicarboxylates to a polymerisation step. The polymerisation may be performed under conditions common for the production of polyesters. For example, the polymerisation may be performed under subatmospheric pressure, such as for example at a pressure of ≤ 1.0 kPa, more preferably ≤ 0.5 kPa, or even more preferred ≤ 0.1 kPa. Such pressure contributes to driving the equilibrium of the polymerisation reaction towards the formation of the polymer.

The polymerisation may for example be performed place at a temperature in the range of ≥ 200 and ≤ 280°C. Preferably, the temperature is in the range of ≥ 220 and ≤ 270°C, or ≥ 230 and ≤ 260°C.

It is preferred that the polymerisation reaction is performed for a period in the range of 30 minutes to 2 hours.

The process for production of polyester may further comprise additional steps, such as a solid state polymerisation step (SSP) subsequent to the polymerisation step. SSP may for example be performed in a vessel such as a tumble drier. To conduct the SSP, the contents of the tumble drier may be subjected to a temperature of for example 120-200°C, preferably 150-180°, for a period of 1-5 hrs, preferably 1-3 hours, to remove moisture, under continuous agitation and under continuous nitrogen flow. Subsequently the SSP reaction may be performed by increasing the temperature to 200-220°C. Preferably, the SSP is performed under a vacuum of ≤ 20 mbar, more preferably ≤ 10 mbar, even more preferably ≤ 5 mbar, under continuous agitation. SSP may for example be performed for a period of 5-50 hours, preferably 10-40 hours, to obtain a polyester.

It is preferred that the polyester that is obtained has an intrinsic viscosity of ≥ 0.45 dl/g, more preferably ≥ 0.50 dl/g, even more preferably ≥ 0.60 dl/g or ≥ 0.80 dl/g. Preferably, the polyester has an intrinsic viscosity of ≤ 2.20 dl/g, more preferably ≤ 2.00 dl/g, even more preferably ≤ 1.50 dl/g. For example, the polyester may have an intrinsic viscosity of ≥ 0.45 and ≤ 2.20 dl/g, preferably ≥ 0.60 and ≤ 2.00 dl/g, more preferably ≥ 0.80 and ≤ 1.50 dl/g. The intrinsic viscosity of the polyester may be determined in accordance with ASTM D2857-95 (2007).

The polyester may for example have a carboxylic end group content of ≤ 50 mmol/g, more preferably ≤ 20 mmol/g. The carboxylic end group content may be determined in accordance with ASTM D7409-15.

In a particular embodiment, the present invention relates to a process for the production of bis-hydroxyalkylene dicarboxylates comprising the steps in this order of:
(a) providing to a reaction vessel a reaction mixture comprising a dicarboxylic acid or a diester thereof and an alkylene diol;
(b) providing a quantity of a catalyst to the reaction mixture; and
(c) reacting the reaction mixture at a temperature in the range of ≥ 200 and ≤ 280°C
wherein the catalyst is a compound according to the formula (I): 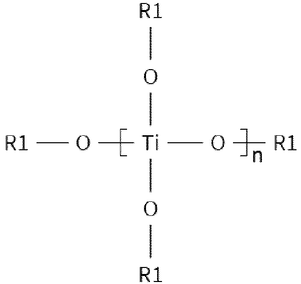 in which R1 is an alkyl moiety comprising 2-10 carbon atoms and n is an integer of ≥ 2 wherein
- the dicarboxylic acid or diester thereof is terephthalic acid, dimethylterephthalate or 2,5furandicarboxylic acid; and
- the alkylene diol is ethylene glycol or 1,4-butanediol.

A further embodiment of the invention relates to a process for the production of bis-hydroxyalkylene dicarboxylates comprising the steps in this order of:
(a) providing to a reaction vessel a reaction mixture comprising a dicarboxylic acid or a diester thereof and an alkylene diol;
(b) providing a quantity of a catalyst to the reaction mixture; and
(c) reacting the reaction mixture at a temperature in the range of ≥ 200 and ≤ 280°C
wherein the catalyst is a compound according to the formula (I): 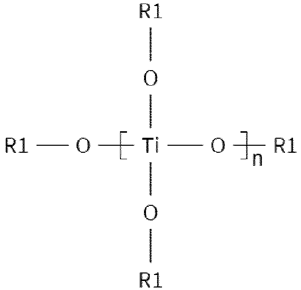 in which R1 is an alkyl moiety comprising 2-10 carbon atoms and n is an integer of ≥ 2 wherein
- the dicarboxylic acid or diester thereof is terephthalic acid, dimethylterephthalate or 2,5-furandicarboxylic acid;
- the alkylene diol is ethylene glycol or 1,4-butanediol; and
- the catalyst is selected from poly(tetraethyl titanate), poly(tetrabutyl titanate), or poly(tetraisobutyl titanate).

In yet a further embodiment, the invention relates to a process for the production of bis-hydroxyalkylene dicarboxylates comprising the steps in this order of:
(a) providing to a reaction vessel a reaction mixture comprising a dicarboxylic acid or a diester thereof and an alkylene diol;
(b) providing a quantity of 50 - 1000 ppm of a catalyst with regard to the total weight of the dicarboxylic acid or diester thereof and the alkylene diol to the reaction mixture; and
(c) reacting the reaction mixture at a temperature in the range of ≥ 200 and ≤ 280°C
wherein the catalyst is a compound according to the formula (I): 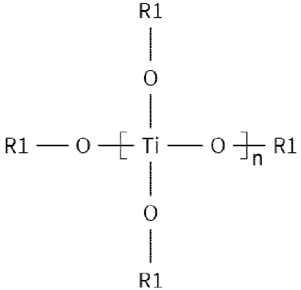 in which R1 is an alkyl moiety comprising 2-10 carbon atoms and n is an integer of ≥ 2
wherein
- the dicarboxylic acid or diester thereof is terephthalic acid, dimethylterephthalate or 2,5-furandicarboxylic acid;
- the alkylene diol is ethylene glycol or 1,4-butanediol; and
- the catalyst is selected from poly(tetraethyl titanate), poly(tetrabutyl titanate), or poly(tetraisobutyl titanate).

Another particular embodiment of the invention relates to a process for the production of bis-hydroxyalkylene dicarboxylates comprising the steps in this order of:
(a) providing to a reaction vessel a reaction mixture comprising a dicarboxylic acid selected from terephthalic acid or 2,5-furandicarboxylic acid and an alkylene diol selected from ethylene glycol or 1,4-butanediol;
(b) providing a quantity of 50 - 1000 ppm of a catalyst with regard to the total weight of the dicarboxylic acid and the alkylene diol to the reaction mixture; and
(c) reacting the reaction mixture at a temperature in the range of ≥ 200 and ≤ 280°C
wherein the catalyst is a compound selected from poly(tetraethyl titanate), poly(tetrabutyl titanate), or poly(tetraisobutyl titanate).

A further particularly preferable embodiment of the invention relates to a process for the production of bis-hydroxyalkylene dicarboxylates comprising the steps in this order of:
(a) providing to a reaction vessel a reaction mixture comprising a dicarboxylic acid or a diester thereof and an alkylene diol wherein the molar ratio of the alkylene diol to the dicarboxylic acid or diester thereof is ≥ 2.0 and ≤ 3.0;
(b) providing a quantity of 50 - 1000 ppm of a catalyst with regard to the total weight of the dicarboxylic acid or diester thereof and the alkylene diol to the reaction mixture; and
(c) reacting the reaction mixture at a temperature in the range of ≥ 200 and ≤ 280°C
wherein the catalyst is a compound according to the formula (I): 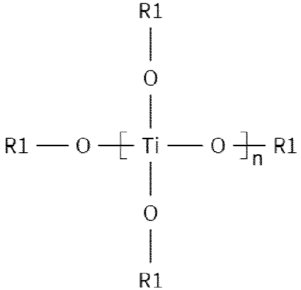 in which R1 is an alkyl moiety comprising 2-10 carbon atoms and n is an integer of ≥ 2
wherein
- the dicarboxylic acid or diester thereof is terephthalic acid, dimethylterephthalate or 2,5-furandicarboxylic acid;
- the alkylene diol is ethylene glycol or 1,4-butanediol; and
- the catalyst is selected from poly(tetraethyl titanate), poly(tetrabutyl titanate), or poly(tetraisobutyl titanate).

More particularly preferred, the invention further relates to a process for the production of bis-hydroxyalkylene dicarboxylates comprising the steps in this order of:
(a) providing to a reaction vessel a reaction mixture comprising a terephthalic acid and 1,4-butanediol wherein the molar ratio of the 1,4-butanediol to the terephthalic acid is ≥ 2.0 and ≤ 3.0;
(b) providing a quantity of 50 - 1000 ppm of a catalyst with regard to the total weight of the dicarboxylic acid or diester thereof and the alkylene diol to the reaction mixture; and
(c) reacting the reaction mixture at a temperature in the range of ≥ 200 and ≤ 280°C
wherein the catalyst is selected from poly(tetraethyl titanate), poly(tetrabutyl titanate), or poly(tetraisobutyl titanate).

More particularly preferred, the invention further relates to a process for the production of bis-hydroxyalkylene dicarboxylates comprising the steps in this order of:
(a) providing to a reaction vessel a reaction mixture comprising a terephthalic acid and 1,4-butanediol wherein the molar ratio of the 1,4-butanediol to the terephthalic acid is ≥ 2.0 and ≤ 3.0;
(b) providing a quantity of 50 - 1000 ppm of a catalyst with regard to the total weight of the dicarboxylic acid or diester thereof and the alkylene diol to the reaction mixture; and
(c) reacting the reaction mixture at a temperature in the range of ≥ 200 and ≤ 280°C
wherein the catalyst is a poly(tetrabutyl titanate).

The invention will now be illustrated by the following non-limiting examples.

A number of experiments were performed demonstrating the advantageous effects of the process of the present invention. The experiments involved the synthesis of bis-hydroxyalkylene dicarboxylates and the polymerisation thereof to polyesters. The materials used in the experiments are listed in the table below.

**Table I: Materials**

| | |
|---|---|
| BDO | 1,4-Butanediol, 99.5% purity, obtainable from BASF |
| PTA | Terephthalalic acid, 99%+ purity, obtainable from British Petroleum |
| TPT | Tetraisopropyl titanate, CAS reg. nr. 546-68-9, obtainable from Dorf Ketal |
| TBT | Tetrabutyl titanate, CAS reg. nr. 5593-70-4, obtainable from Sigma Aldrich |
| PTBT | Polymeric tetrabutyl titanate, CAS reg. nr. 9022-96-2, obtainable from Fisher |

### Example 1: Preparation of bis-hydroxybutylene terephthalate using PTBT catalyst.

A quantity of 74.8 g BDO and 121.7 g PTA were introduced into a 3-neck round bottom flask having a volume of 1 I. The flask was placed in an oil bath were the temperature of the oil was set at 240°C. The contents of the flask were allowed to adopt the temperature of the oil bath for 10 minutes. 180 ppm of PTBT was added to the reactor to initiate the reaction. The temperature was maintained at 240°C and the flask was stirred at 260 rpm under nitrogen atmosphere at 1 bar pressure. The reaction was allowed to continue until the clearing point was reached, where the time between feed of the catalyst and the reaching of the clearing point was determined. The clearing point is to be understood to be reached when the reaction medium becomes homogeneous, which is when the reaction medium becomes a clear liquid. At that point, no unreacted PTA remained in the reaction mixture. A reaction mixture comprising the bis-hydroxybutylene terephthalate was obtained. In example 1, the clearing point was reached after 27 minutes.

### Example 2: Preparation of bis-hydroxybutylene terephthalate using TPT catalyst.

A quantity of 74.8 g BDO and 121.7 g PTA were introduced into a 3-neck round bottom flask having a volume of 1 I. The flask was placed in an oil bath were the temperature of the oil was set at 240°C. The contents of the flask were allowed to adopt the temperature of the oil bath for 10 minutes. 180 ppm of TPT was added to the reactor to initiate the reaction. The temperature was maintained at 240°C and the flask was stirred at 260 rpm under nitrogen atmosphere at 1 bar pressure. The reaction was allowed to continue until the clearing point was reached, where the time between feed of the catalyst and the reaching of the clearing point was determined. The clearing point is to be understood to be reached when the reaction medium becomes homogeneous, which is when the reaction medium becomes a clear liquid. At that point, no unreacted PTA remained in the reaction mixture. A reaction mixture comprising the bis-hydroxybutylene terephthalate was obtained. In example 2, the clearing point was reached after 34 minutes.

### Example 3: Preparation of bis-hydroxybutylene terephthalate using TBT catalyst.

A quantity of 74.8 g BDO and 121.7 g PTA were introduced into a 3-neck round bottom flask having a volume of 1 I. The flask was placed in an oil bath were the temperature of the oil was set at 240°C. The contents of the flask were allowed to adopt the temperature of the oil bath for 10 minutes. 180 ppm of TPT was added to the reactor to initiate the reaction. The temperature was maintained at 240°C and the flask was stirred at 260 rpm under nitrogen atmosphere at 1 bar pressure. The reaction was allowed to continue until the clearing point was reached, where the time between feed of the catalyst and the reaching of the clearing point was determined. The clearing point is to be understood to be reached when the reaction medium becomes homogeneous, which is when the reaction medium becomes a clear liquid. At that point, no unreacted PTA remained in the reaction mixture. A reaction mixture comprising the bis-hydroxybutylene terephthalate was obtained. In example 3, the clearing point was reached after 31 minutes.

### Example 4: Preparation of PBT using the reaction mixture of example 1.

Upon reaching the clearing point in the synthesis of example 1, the flask was subjected to a vacuum of 0.2 mbar. Polymerisation started using the reaction mixture obtained in example 1, under the same temperature of 240°C and stirring at 260 rpm as was the case in example 1. Polymerisation was allowed to continue until a polymer build of 4 was reached. The polymerisation time was 31 minutes.

### Example 5: Preparation of PBT using the reaction mixture of example 2.

Upon reaching the clearing point in the synthesis of example 2, the flask was subjected to a vacuum of 0.2 mbar. Polymerisation started using the reaction mixture obtained in example 2, under the same temperature of 240°C and stirring at 260 rpm as was the case in example 2. Polymerisation was allowed to continue until a polymer build of 4 was reached. The polymerisation time was 38 minutes.

### Example 6: Preparation of PBT using the reaction mixture of example 3.

Upon reaching the clearing point in the synthesis of example 3, the flask was subjected to a vacuum of 0.2 mbar. Polymerisation started using the reaction mixture obtained in example 3, under the same temperature of 240°C and stirring at 260 rpm as was the case in example 3. Polymerisation was allowed to continue until a polymer build of 4 was reached. The polymerisation time was 39 minutes.

The above examples demonstrate the advantages of the process on the invention, reflected by examples 1 and 4, in that both the duration of preparation of the bis-hydroxyalkylene dicarboxylates and the subsequent polycondensation to obtain a polyester using the reaction product of the preparation of the bis-hydroxyalkylene dicarboxylates are shorter.

## Claims

1. Process for the production of bis-hydroxyalkylene dicarboxylates comprising the steps in this order of:
(a) providing to a reaction vessel a reaction mixture comprising a dicarboxylic acid or a diester thereof and an alkylene diol;
(b) providing a quantity of a catalyst to the reaction mixture; and
(c) reacting the reaction mixture at a temperature in the range of ≥ 180 and ≤ 280°C
wherein the catalyst is a compound according to the formula (I): 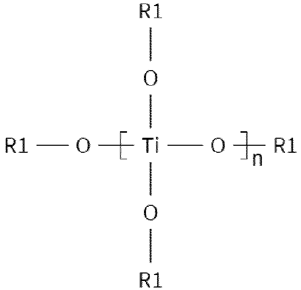 in which R1 is an alkyl moiety comprising 2-10 carbon atoms and n is an integer of ≥ 2.

2. Process according to claim 1, wherein n is an integer of ≥ 4 and ≤ 100.

3. Process according to any one of claims 1-2, wherein the reaction in step (c) is performed at a temperature of ≥ 180 °C and ≤ 260°C and a pressure of ≤ 101 kPa.

4. Process according to any one of claims 1-3, wherein each R1 is the same and selected from ethyl, propyl, isopropyl, butyl or isobutyl.

5. Process according to any one of claims 1-4 wherein the catalyst is selected from poly(tetraethyl titanate), poly(tetrabutyl titanate), or poly(tetraisopropyl titanate).

6. Process according to any one of claims 1-5 wherein the catalyst is provided to the reaction mixture in a quantity of 50 - 1000 ppm by weight with regard to the total weight of the dicarboxylic acid or diester thereof and the alkylene diol in the reaction mixture.

7. Process according to any one of claims 1-6 wherein the dicarboxylic acid or diester thereof is an aromatic dicarboxylic acid or diester thereof.

8. Process according to any one of claims 1-7 wherein the dicarboxylic acid or diester thereof comprises 4-20 carbon atoms.

9. Process according to any one of claims 1-8 wherein the dicarboxylic acid or diester thereof is selected from terephthalic acid, dimethyl terephthalate, isophthalic acid, or 2,5-furandicarboxylic acid.

10. Process according to any one of claims 1-9 wherein the alkylene diol comprises 2-10 carbon atoms.

11. Process according to any one of claims 1-10 wherein the alkylene diol is selected from ethylene glycol, propanediol, 1,4-butanediol, or cyclohexanedimethanol.

12. Process according to any one of claims 1-11 wherein the molar ratio of the alkylene diol to the dicarboxylic acid or diester thereof in the reaction mixture is ≥ 1.1.

13. Process for production of a polyester comprising the reaction product comprising bis-hydroxyalkylene dicarboxylates obtained according to any one of claims 1-12.

14. Process according to claim 13 wherein the polyester is poly(ethylene terephthalate), poly(ethylene naphthalate), poly(ethylene furanoate), or poly(butylene terephthalate).

## Patentansprüche

1. Vorgang für die Herstellung von Bishydroxyalkylendicarboxylaten, umfassend in dieser Reihenfolge die Schritte von:
(a) Bereitstellen an ein Reaktionsgefäß einer Reaktionsmischung, die Dicarbonsäure oder einen Diester davon und ein Alkylendiol umfasst;
(b) Bereitstellen einer Menge eines Katalysators an die Reaktionsmischung; und
(c) Reagieren der Reaktionsmischung bei einer Temperatur in dem Bereich von ≥180 und ≤280 °C,
wobei der Katalysator eine Verbindung gemäß der Formel (I) ist: 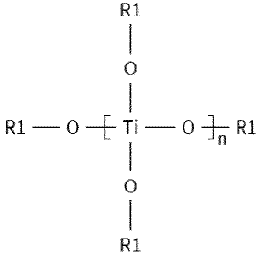 in der R1 eine Alkyleinheit ist, die 2-10 Kohlenstoffatome umfasst, und n eine ganze Zahl von ≥2 ist.

2. Vorgang nach Anspruch 1, wobei n eine ganze Zahl von ≥4 und ≤100 ist.

3. Vorgang nach einem der Ansprüche 1-2, wobei die Reaktion in Schritt (c) bei einer Temperatur von ≥180 °C und ≤260 °C und einem Druck von ≤101 kPa durchgeführt wird.

4. Vorgang nach einem der Ansprüche 1-3, wobei jedes R1 dasselbe ist und aus Ethyl, Propyl, Isopropyl, Butyl oder Isobutyl ausgewählt wird.

5. Vorgang nach einem der Ansprüche 1-4, wobei der Katalysator aus Poly(tetraethyltitanat), Poly(tetrabutyltitanat) oder Poly(tetraisopropyltitanat) ausgewählt wird.

6. Vorgang nach einem der Ansprüche 1-5, wobei der Katalysator der Reaktionsmischung in einer Menge von 50-1000 Gewicht-ppm in Bezug zu dem Gesamtgewicht der Dicarbonsäure oder dem Diester davon und dem Alkylendiol in der Reaktionsmischung bereitgestellt wird.

7. Vorgang nach einem der Ansprüche 1-6, wobei die Dicarbonsäure oder der Diester davon eine aromatische Dicarbonsäure oder ein aromatischer Diester davon ist.

8. Vorgang nach einem der Ansprüche 1-7, wobei die Dicarbonsäure oder der Diester davon 4-20 Kohlenstoffatome umfasst.

9. Vorgang nach einem der Ansprüche 1-8, wobei die Dicarbonsäure oder der Diester davon aus Terephthalsäure, Dimethylterephthalat, Isophthalsäure oder 2,5-Furandicarbonsäure ausgewählt wird.

10. Vorgang nach einem der Ansprüche 1-9, wobei das Alkylendiol 2-10 Kohlenstoffatome umfasst.

11. Vorgang nach einem der Ansprüche 1-10, wobei das Alkylendiol aus Ethylenglycol, Propandiol, 1,4-Butandiol oder Cyclohexandimethanol ausgewählt wird.

12. Vorgang nach einem der Ansprüche 1-11, wobei das Molverhältnis des Alkylendiols zu der Dicarbonsäure oder dem Diester davon in der Reaktionsmischung ≥1,1 beträgt.

13. Vorgang für die Herstellung eines Polyesters, umfassend das Reaktionsprodukt, das Bis-hydroxyalkylendicarboxylate umfasst, die nach einem der Ansprüche 1-12 erhalten wurden.

14. Vorgang nach Anspruch 13, wobei der Polyester Poly(ethylenterephthalat), Poly(ethylennaphthalat), Poly(ethylenfuranoat) oder Poly(butylenterephthalat) ist.

## Revendications

1. Procédé de production de dicarboxylates de bis-hydroxyalkylène comprenant les étapes dans cet ordre consistant à :
(a) fournir à un récipient réactionnel un mélange réactionnel comprenant un acide dicarboxylique ou un diester de celui-ci et un alkylène diol ;
(b) fournir une quantité d'un catalyseur au mélange réactionnel ; et
(c) faire réagir le mélange réactionnel à une température dans la plage ≥ 180 et ≤ 280°C, dans lequel le catalyseur est un composé selon la formule (I) : 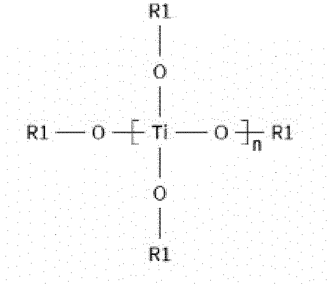 dans laquelle R1 est un fragment alkyle comprenant 2 à 10 atomes de carbone et n est un nombre entier ≥ 2.

2. Procédé selon la revendication 1, dans lequel n est un nombre entier ≥ 4 et ≤ 100.

3. Procédé selon l'une quelconque des revendications 1 à 2, dans lequel la réaction à l'étape (c) est effectuée à une température ≥ 180°C et ≤ 260°C et une pression ≤ 101 kPa.

4. Procédé selon l'une quelconque des revendications 1 à 3, dans lequel chaque R1 est identique et choisi parmi l'éthyle, le propyle, l'isopropyle, le butyle ou l'isobutyle.

5. Procédé selon l'une quelconque des revendications 1 à 4, dans lequel le catalyseur est choisi parmi le poly(titanate de tétraéthyle), le poly(titanate de tétrabutyle) ou le poly(titanate de tétraisopropyle).

6. Procédé selon l'une quelconque des revendications 1 à 5, dans lequel le catalyseur est fourni au mélange réactionnel en une quantité de 50 à 1000 ppm en poids par rapport au poids total de l'acide dicarboxylique ou de son diester et de l'alkylène diol dans le mélange réactionnel.

7. Procédé selon l'une quelconque des revendications 1 à 6, dans lequel l'acide dicarboxylique ou son diester est un acide dicarboxylique aromatique ou son diester.

8. Procédé selon l'une quelconque des revendications 1 à 7, dans lequel l'acide dicarboxylique ou son diester comprend 4 à 20 atomes de carbone.

9. Procédé selon l'une quelconque des revendications 1 à 8, dans lequel l'acide dicarboxylique ou son diester est choisi parmi l'acide téréphtalique, le téréphtalate de diméthyle, l'acide isophtalique ou l'acide 2,5-furandicarboxylique.

10. Procédé selon l'une quelconque des revendications 1 à 9, dans lequel l'alkylène diol comprend 2 à 10 atomes de carbone.

11. Procédé selon l'une quelconque des revendications 1 à 10, dans lequel l'alkylène diol est choisi parmi l'éthylène glycol, le propanediol, le 1,4-butanediol ou le cyclohexanediméthanol.

12. Procédé selon l'une quelconque des revendications 1 à 11, dans lequel le rapport molaire de l'alkylène diol par rapport à l'acide dicarboxylique ou à son diester dans le mélange réactionnel est ≥ 1,1.

13. Procédé de production d'un polyester comprenant le produit réactionnel comprenant les dicarboxylates de bis-hydroxyalkylène obtenus selon l'une quelconque des revendications 1 à 12.

14. Procédé selon la revendication 13, dans lequel le polyester est le poly(téréphtalate d'éthylène), le poly(naphtalate d'éthylène), le poly(furanoate d'éthylène) ou le poly(téréphtalate de butylène).
